# EUROPEAN PATENT SPECIFICATION

(11) **EP 1 234 018 B1**
(45) Date of publication and mention of the grant of the patent: **31.03.2004**
(21) Application number: 00963219.1
(22) Date of filing: 07.09.2000
(51) Int. Cl.: C12M 1/42, C12N 13/00

(54) **Method for porating biological membranes**
Verfahren zur Poration biologischer Membranen
Procédé de poration des membranes biologiques

(30) Priority: 08.09.1999 SE 9903183; 08.09.1999 SE 9903185; 08.09.1999 SE 9903187
(43) Date of publication of application: 28.08.2002
(73) Proprietor: Genovis AB, 223 70 Lund (SE)
(72) Inventor: FREDRIKSSON, Sarah, S-245 61 Staffanstorp (SE); KRIZ, Dario, S-243 95 Höör (SE)
(74) Representative: Bjerre, Nils B.J.
(86) International application number: PCT/SE2000/001743
(87) International publication number: WO 2001/018168

(56) References cited:
- TIMOTHY E. VAUGHAN ET AL.: 'Energetic constraints on the creation of cell membrane pores by magnetic particles' BIOPHYSICAL JOURNAL vol. 71, August 1996, pages 616 - 622, XP002935320
- TIMOTHY E. VAUGHAN ET AL.: 'Molecular change due to biomagnetic stimulation and transient magnetic fields: Mechanical interference constraints on possible effects by cell membrane pore creation via magnetic particles' BIOELECTROCHEMISTRY AND BIOENERGETICS vol. 46, 1998, pages 121 - 128, XP002935319

## Description

### Field of the Invention

The present invention relates to a method for use in, inter alia, molecular biological work.

### Background Art

In the fields of research biotechnology and bio-medicine, it is often necessary to introduce a large molecule or a bioparticle into a biological structure, such as a bacterial cell. Cells and also viruses have an outer barrier for protection against the environment and also a selective transport system for nutritive substances. In order to force natural protective mechanisms and introduce a substance which is not desirable for the target organism, some sort of chemical or physical treatment of the target cell is necessary. Examples of techniques of forcing the outer cell membrane of cells, and where appropriate also the cell wall, are available in the fields of research genetic engineering and molecular biology.

When a new genetic code is transferred to a specially selected host cell, the technique is referred to as transformation or transfection. There is no general method to be used for all types of cells, but a technique is available for each cell type and purpose. Moreover it is not possible to transform all cell types using the techniques that are currently available. In 1970, Mandel and Higa (*J. Mol. Bio.* 53: 159-162) reported that *E.coli* cells which had been pre-treated with CaCl₂ could be made to take up foreign DNA when subjected to a temperature shock. After that the method has been continuously developed, (see e.g. WO9728248). By exposing cells, during a fraction of a second, to an electric pulse of high voltage, pores in cell membranes open, referred to as electroporation (Zimmerman et al. *J. Membr. Biol. 67*: 165-82 (1983)), which is frequently used as transformation technique. Bacteria, yeast and in some cases also mammalian cells and plant cells can, in specific conditions, be transformed by means of electroporation. Also in this case, a continuous development of the technique is in progress (see WO9812310 and WO9906101). In the two methods described above, the cell envelope is opened sufficiently long for the DNA mclecule to enter the cell. The third and last developed method for transformation is so-called lipofection (Old and Primrose, in *Principles of Gene Manipulation: An Introduction to Gene Manipulation,* Blackwell Science (1995)) where the foreign DNA is enclosed in/binds to a cationic liposome which fuses with the outer membrane of the target cell. There is one more commercial technique for transformation of plant cells, where a plant part selected for the purpose is bombarded with small gold grains which are prepared with the foreign gene (Boynton J.E. et. Science 240, 1534-1538, 1988). Such gene transfer has been developed for transformation of other tissues, such as bacteria, fungi, insect and mammalian cells (Johnston S.A. Nature 346, 776-777, 1990).

A further method for creating transient pores in biological membranes is decribed by T. E. Vaughan & J. C. Weaver (see Biophys. J. (1996) 71, 616-622 and Bioelectrochem. Bioenerg. (1998) 46, 121-128) which is based on the application of a pulsed magnetic field to biological cells or tissues comprising naturally-ocurring magnetic particles.

It is especially in the applications described above that the present invention can be used. However, it is quite possible to use the inventive device to introduce other exogenic materials in applications, such as direct transfer of proteins, RNA molecules, fatty acids, peptides, medical preparations etc., to study the response of specific cells and viruses. Moreover the device according to the invention is particularly suitable for lysis of cells for the purpose of carrying out lysis as well as identification and isolation of specific cellular components in one and the same method.

### Summary of the Invention

Thus, the present invention relates to a method for introduction and/or extraction of particles into/from biological membrane - enveloped structures as defined in the appended claims. The device used is characterised in that it comprises at least one coil in which a magnetic alternating field can be generated and into which a sample can be inserted, where said magnetic field causes an increase of the thermal and/or kinetic energy of magnetically susceptible particles in said sample, the increased thermal and/or kinetic energy of said particles causing the formation of pores in biological membrane-enveloped structures which are to be found in said sample, said pores allowing introduction or extraction of bioparticles into/from said biological membrane-enveloped structures.

The method according to the invention is used for specific lysis of cells, or to modify the genetic code and/or metabolism of a host cell.

### Brief Description of the Drawings

Fig. 1 is a principle sketch of the device according to the present invention.
Fig. 2 is an Example of an electronic current supply circuit.
Fig. 3 is an Example of the connection of a coil.
Fig. 4 shows an Example of a magnetically susceptible particle.
Fig. 5 shows a device which can generate a gradient field.

### Detailed Description of the Invention

According to one aspect of the invention, the method is characterised in that said magnetic field has an alternating field direction of a frequency in the range 1-5 MHz.

According to another aspect, the method is characterised in that said magnetic field has a field strength of at least 1 mT.

According to one more aspect, the invention is characterised in that said magnetic field is non-homogeneous and has an alternating gradient field direction, the direction of said alternating gradient field being generated by two coils, and said sample is inserted between the coils.

According to one more aspect, the method according to the invention is characterised in that said coils are supplied with alternating current of different frequencies.

According to yet another aspect, the method is characterised in that said coils are supplied with either the positive or the negative part of the supplied alternating current.

According to another aspect, the method involves the use of a thermostat for accurate temperature control of said coil or coils and/or said sample.

According to a further aspect, the method requires a variable timing for accurate control of the time during which said alternating current is on and during which said sample is exposed to said applied magnetic field.

According to another aspect, the method involves the use of a control system for accurate setting of strength and frequency of said alternating current.

The biological membrane-enveloped structures consist of, inter alia, body tissue, cells, bacteria, virus particles, organelles at a sub-cellular level, liposomes or proteins.

The bioparticles that are suitable for introduction into/extraction from membrane-enveloped structures are, inter alia, DNA molecules, RNA molecules, proteins, other biopolymers, peptides, chemical preparations, organic compounds, inorganic compounds or synthetic polymers or combinations thereof.

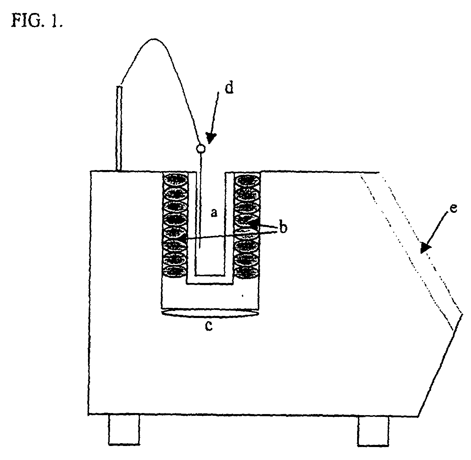

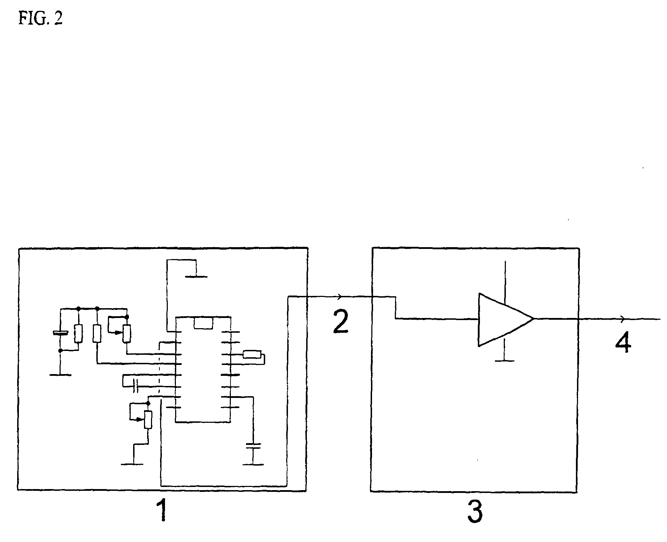

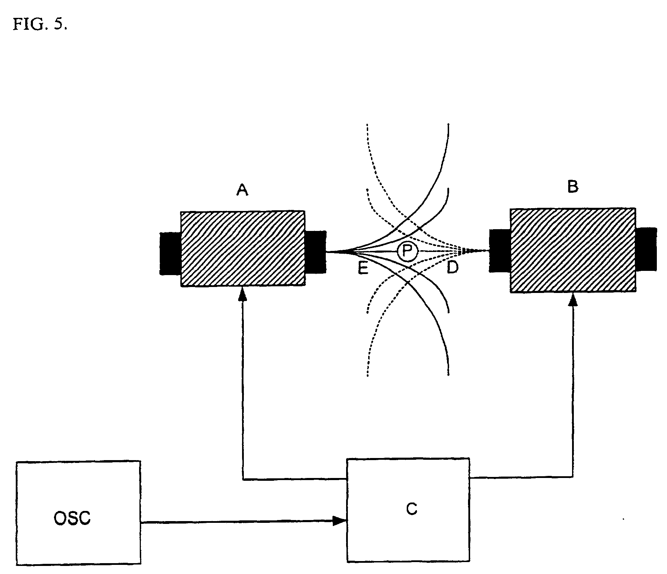

The technique on which the invention is partly based is a combination of magnetic nanotechnology and peptide chemistry. A magnetically susceptible particle having a size of between some ten micrometers and one nanometer is used as a reagent in the technique. When such a particle is exposed to a certain magnetic field, it is made to vibrate and generate heat. Fig. 1 is a principle sketch of the present invention. The biological sample is mixed with a reagent intended for the purpose and is then placed in a sample holder (a). The desired.strength and frequency of the magnetic source (b) are adjusted, whereupon the desired temperature of the cooling element (c) is adjusted. The magnetic source is either one coil or two coils, the sample being placed between them according to Fig. 5. The magnetic source and the sample holder are enclosed in an isolated unit, in which the temperature is determined by the cooling element. To ensure the correct temperature in the sample holder, a temperature sensor (d) can be connected to the system. The variables temperature, strength and frequency of the magnetic field and treatment intervals are controlled and can be followed on a digital display (e).

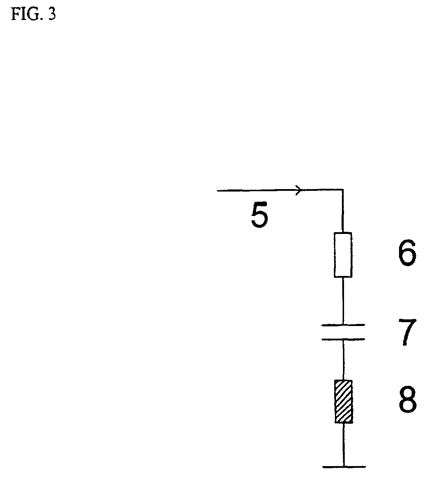

Fig. 2 illustrates an example of en electronic current supply circuit which comprises an oscillator (1) based on the circuit XR2206, whose output signal (2) is amplified by a power amplifying step (3), which is based on the circuit PBD 3548/1, whose output signal (4) can operate an alternating current (1MHz, 2A) through one or more coils. An example of the connection of said coil is shown in Fig. 3, with an oscillating circuit consisting of a 2 Ω resistance (6), a 0.50 nF capacitor (7) and a 50 µH coil (8), said circuit being supplied with alternating current (5). For a person skilled in the art it is obvious that the above-described example illustrated in Figs 2 and 3 can easily be modified and that the same result can be obtained with the aid of alternative connections and coils.

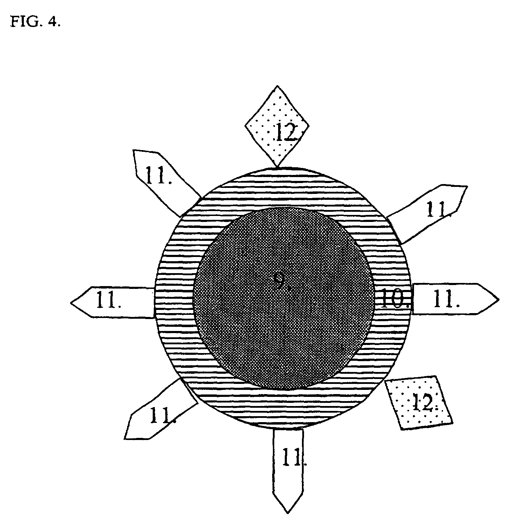

Examples of magnetic materials that are used in the method according to the invention are described in the patent literature, e.g. US 4,323,056 (*Borelli et al*). The magnetically susceptible particle and a possible configuration are also illustrated in Fig. 4. The magnetically susceptible core (9) of the particle consists essentially of magnetite (iron oxide). Further the particle is coated with an outer layer (10) consisting of a derivatised polymer (Dextran), or a monolayer or alternatively bilayer of derivatised fatty acids. The choice of the type (number of amino acid or carbohydrate units and sequence) of ligand 11 which is used for the derivatisation is individually adapted to each application of the magnetically susceptible particle, the effect of which can be still more amplified by its surface being further modified with one or more effector molecules 12.

By adding said particles to a cell suspension and then exposing the cell to a magnetic field with alternating field direction, instantaneous heating of the medium surrounding each magnetically susceptible particle is obtained. The heat induces a temperature shock in cell and cell membrane, which causes temporary openings in the cell membrane. The heat is induced quickly and homogeneously in the entire sample, which makes it possible for the sample and the cells to be exposed to treatment for a short while, which increases the survival frequency of the exposed cells. Example 1 describes transformation of *Escherichia coli*.

In a conventional transformation method involving a temperature shock, the test tube containing the cell suspension is exposed to a higher ambient temperature (42°C), whereby a temperature gradient arises from the test tube wall and into the sample, the composition of which requires a longer time than the method according to the present invention and which further implies that the cells that are located closest to the cell wall are exposed to the higher temperature for a longer time than those in the centre of the tube. Thus, some of the cells will die owing to the increased temperature while a fraction of the cells remain untreated. The method according to the invention circumvents this problem by the instantaneous heating round each particle in the sample holder. The effect is amplified if the particle is besides directed immediately to the cell envelopes via the ligand molecules on the surface of the particle. This is a great advantage compared with conventional transformation methods where the balance between heat shock and cells death is important to the final result.

Furthermore, the field strength of the magnetic field can be varied in space, a so-called gradient field which in combination with alternating field direction causes mechanical vibrations (kinetic energy increases) in the particles, which in combination with heat radiation (thermal energy) amplifies the effect of the particles on the cell membrane surrounding all cells (and cell wall, where appropriate). The present invention describes a completely new method involving induction of heat or powerful introduction of shearing forces, or a combination thereof. The shearing forces initiate dislocations in the cell membrane owing to mechanical fatigue, which results in breaks in cell membranes (and cell walls in the cases where the target cell is, for example, a bacterium). The method is based on the use of an alternating externally applied gradient magnetic field. A gradient field is provided with at least two coils, which are supplied with either the positive or the negative part of the supplied alternating current, or alternatively said coils are supplied with alternating current of different frequencies. A device which can generate a gradient field is described in Fig. 5. The functional principle is based on two coils (A) and (B) (with or without ferrite core) being arranged opposite to each other according to Fig. 5. A control unit (C) controls the current through the coils, so that the coils only one at a time have a current passing through their windings. This current alternation, whose frequency is controlled by means of the oscillator (OSC), results in the coils alternatingly generating the gradient magnetic fields (D) and (E) with different gradient directions. A magnetically susceptible particle (P) located between the coils will experience a gradient magnetic field with periodically alternating direction, which will induce a mechanical vibration. Alternatively, a gradient field can be generated when the two coils are supplied with current of two different frequencies. The difference in frequency between the current in the two coils controls the frequency of the alternation of the gradient.

By letting the magnetic treatment take place for a short while, conditions are created for a large number of surviving cells after treatment. As long as the cell envelope is open, the molecule which is to be transformed should be introduced into the cell. To optimise this procedure, the molecule can also be directed to the cell envelope. Both processes are effected by connecting recognition molecules for binding on the one hand to the cell surface and, on the other hand, to said molecule of one and the same ferromagnetic particle. Molecules, which on a biochemical basis can recognise and bind to biological structures of different kinds can be, for example, short synthetic peptides, parts of an antibody or an enzyme.

By connecting a recognition molecule of a target protein, such a recombinant protein, to the magnetically susceptible particles, the device according to the invention can be used for lysis and specific purification of said target protein in one and the same method. Compared with alternative techniques of lysis (mainly enzymatic and mechanical lysis), in combination with one or more purification steps, the use of the device according to the invention saves above all time, but also material.

The inventive device can advantageously be used on the one hand for a transformation method and, on the other hand, for purification of specific cell components, which makes the device unique. Regardless of the purpose, the method should take place while the coils are kept at a constant temperature, which means that a cooling element and a temperature control should be incorporated into the controllable magnetic equipment. Moreover it is advantageous for the various potential fields of application of the device that the strength and frequency of the magnetic field as well as the time during which the sample is exposed to the treatment are variable.

### EXAMPLE 1

The following Example describes a method for transformation of *Escherichia coli (E.coli)* with pUC18 plasmid:

100 µl competent *E.coli* cells are mixed at 0°C with 500 µg pUC18 dissolved in 30 µl 0.05 M CaCl₂. The sample is introduced into the sample container in the device according to the invention and incubated for 30 min at 0°C in the coil. Then the sample is treated for 30 s at 1MHz, 2A. 1 ml sterile LB broth is then added to the sample, which is then incubated in water bath for 1 h at 37°C. Subsequently the cells are spread on agar plates containing selection pressure, 50 µg/µl ampicillin, for only the transformed bacteria to be obtained. The experiment should include a reference sample which does not contain pUC18 in order to assess survival and transformation frequency.

## Claims

1. A method for introduction or extraction of bioparticles into/from biological membrane-enveloped structures in vitro, comprising:
applying a magnetic alternating field to a sample comprising biological membrane-enveloped structures and magnetically susceptible particles, whereby an increase of the thermal and/or kinetic energy of said magnetically susceptible particles causes the formation of pores in said biological membrane-enveloped structures,
which pores allows the introduction or extraction of bioparticles into/from said biological membrane-enveloped structures.

2. A method according to claim 1, wherein said magnetic field has an alternating field direction of a frequency in the range 1-5 MHz.

3. A method according to claim 1 or 2, wherein said magnetic field has a field strength of 1 mT.

4. A method according to any one of claims 1-3, wherein said magnetic field is non-homogeneous and has an alternating gradient field direction, the direction of said alternating gradient field being generated by two coils, and said sample is inserted between the coils.

5. A method according to claim 4, wherein said coils are supplied with alternating currents of different frequencies.

6. A method according to claim 4, wherein said coils are supplied with either the positive or the negative part of the supplied alternating current.

7. A method according to any one of claims 1-6, wherein said bioparticles are selected from the group comprising DNA molecules, RNA molecules, proteins, other biopolymers, petides, chemical preparations, organic compounds, inorganic compounds or synthetic polymers or combinations thereof.

8. A method according to any one of claims 1-7, wherein said biological membrane-enveloped structures are selected from the group comprising of body tissues, cells, bacteria, virus particles, organelles at a subcellular level, liposomes or proteins.

9. A method according to any one of claims 1-8, for use for specific lysis of cells.

10. A method according to any one of claims 1-8, for use for modifying the genetic code of a host cell and/or metabolism.

## Patentansprüche

1. Verfahren zur Einführung oder Extraktion von Bioteilchen in/aus biologischen membranumhüllten Strukturen in vitro mit:
Anlegen eines alternierenden Magnetfeldes an eine Probe mit biologischen membranumhüllten Strukturen und magnetisch empfänglichen Teilchen, wobei ein Anstieg der thermischen und/oder kinetischen Energie der magnetisch empfänglichen Teilchen die Bildung von Poren in den biologischen membranumhüllten Strukturen verursacht,
wobei die Poren die Einführung oder Extraktion von Bioteilchen in/aus den biologischen membranumhüllten Strukturen ermöglicht.

2. Verfahren nach Anspruch 1, wobei das Magnetfeld eine alternierende Feldrichtung mit einer Frequenz im Bereich von 1-5 MHz hat.

3. Verfahren nach Anspruch 1 oder 2, wobei das magnetische Feld eine Feldstärke von 1 mT hat.

4. Verfahren nach einem der Ansprüche 1-3, wobei das Magnetfeld nicht homogen ist und eine alternierende Gradientenfeldrichtung hat, wobei die Richtung des alternierenden Gradientenfelds durch zwei Spulen erzeugt wird und die Probe zwischen den Spulen eingesetzt wird.

5. Verfahren nach Anspruch 4, wobei die Spulen mit Wechselströmen verschiedener Frequenzen versorgt werden.

6. Verfahren nach Anspruch 4, wobei die Spulen entweder mit dem positiven oder dem negativen Anteil des angelegten Wechselstroms versorgt werden.

7. Verfahren nach einem der Ansprüche 1-6, wobei die Bioteilchen ausgewählt sind aus der Gruppe umfassend DNA-Moleküle, RNA-Moleküle, Proteine, andere Biopolymere, Peptide, chemische Zubereitungen, organische Verbindungen, anorganische Verbindungen oder synthetische Polymere oder Kombinationen davon.

8. Verfahren nach einem der Ansprüche 1-7, wobei die biologischen membranumhüllten Strukturen ausgewählt sind aus der Gruppe umfassend Körpergewebe, Zellen, Bakterien, Viruspartikel, Organellen in einen subzellulären Bereich, Liposome oder Proteine.

9. Verfahren nach einem der Ansprüche 1-8 für die Verwendung zur spezifischen Lyse von Zellen.

10. Verfahren nach einem der Ansprüche 1-8, für die Verwendung zur Modifikation des genetischen Codes und/oder des Metabolismus einer Wirtszelle.

## Revendications

1. Procédé pour l'introduction ou l'extraction des bioparticules dans/depuis les structures biologiques enveloppées par une membrane en vitro, comprenant :
l'application d'un champ magnétique alternatif sur un échantillon comprenant des structures biologiques enveloppées par une membrane et des particules susceptibles magnétiquement, moyennant quoi une augmentation de l'énergie thermique et/ou cinétique desdites particules susceptibles magnétiquement provoque la formation de pores dans lesdites structures biologiques enveloppées par une membrane,
lesquels pores permettent l'introduction ou l'extraction des bioparticules dans/depuis lesdites structures biologiques enveloppées par une membrane.

2. Procédé selon la revendication 1, dans lequel ledit champ magnétique a une direction de champ alternatif d'une fréquence dans la gamme de 1-5 MHz.

3. Procédé selon la revendication 1 ou 2, dans lequel ledit champ magnétique a une résistance de champ de 1 mT.

4. Procédé selon l'une quelconque des revendications 1-3, dans lequel ledit champ magnétique est non homogène et a une direction de champ gradient alternatif, la direction dudit champ gradient alternatif étant générée par deux bobines, et ledit échantillon est inséré entre les bobines.

5. Procédé selon la revendication 4, dans lequel lesdites bobines sont alimentées par des courants alternatifs de fréquences différentes.

6. Procédé selon la revendication 4, dans lequel lesdites bobines sont alimentées par soit la partie positive, soit la partie négative du courant alternatif alimenté.

7. Procédé selon l'une quelconque des revendications 1-6, dans lequel lesdites bioparticules sont choisies dans le groupe comprenant des molécules d'ADN, des molécules d'acide ribonucléique, des protéines, d'autres biopolymères, des peptides, des préparations chimiques, des composés organiques, des composés inorganiques ou des polymères synthétiques ou des combinaisons de ces derniers.

8. Procédé selon l'une quelconque des revendications 1-7, dans lequel lesdites structures biologiques enveloppées par une membrane sont choisies dans le groupe comprenant des tissus cellulaires, des cellules; des bactéries, des particules de virus, des organites à niveau sous cellulaire, des liposomes ou des protéines.

9. Procédé selon l'une quelconque des revendications 1-8, pour une utilisation pour une lyse spécifique de cellules.

10. Procédé selon l'une quelconque des revendications 1-8, pour une utilisation pour modifier le code génétique d'une cellule hôte et/ou métabolisme.
